# EUROPEAN PATENT SPECIFICATION

(11) **EP 1 482 807 B1**
(45) Date of publication and mention of the grant of the patent: **27.08.2008**
(21) Application number: 03712538.2
(22) Date of filing: 11.03.2003
(51) Int. Cl.: A23C 9/123, A23C 19/032, C12N 1/21

(54) **FOOD FERMENTATION WITH LACTIC ACID BACTERIA**
NAHRUNGSMITTELFERMENTIERUNG MIT MILCHSÄUREBAKTERIEN
FERMENTATION D'ALIMENTS AVEC DES BACTERIES D'ACIDE LACTIQUE

(30) Priority: 11.03.2002 GB 0205647
(43) Date of publication of application: 08.12.2004
(73) Proprietor: Heller, Knut, 24109 Kiel (DE)
(72) Inventor: BUDDE-NIEKIEL, Andrea, 25712 Burg (DE); GEIS, Arnold, 24589 Nortof (DE); HELLER, Knut, 24109 Kiel (DE); HASSAN, Mohamed, El-Demerdash, Suez-Canal Univ., Ismallia (EG)
(74) Representative: Biehl, Christian
(86) International application number: PCT/IB2003/001429
(87) International publication number: WO 2003/075669

(56) References cited:
- US-A- 6 153 404
- KOCH, B. ET AL.: "Induced Levels of Heat Shock Proteins in a dnaK Mutant of Lactococcus lactis" JOURNAL OF BACTERIOLOGY, vol. 180, no. 15, August 1998 (1998-08), pages 3873-3881, XP002252991
- MOREL, F. ET AL.: "Improved acid tolerance of a recombinant strain of Escherichia coli expressing genes from the acidophilic bacterium Oenococcus oeni" LETTERS IN APPLIED MICROBIOLOGY, no. 33, 2001, pages 126-130, XP002252992
- DE ANGELIS, M. ET AL.: "The acid-stress response in Lactobacillus sanfranciscensis CB1" MICROBIOLOGY, no. 147, 2001, pages 1863-1873, XP002252993

## Description

### FIELD OF THE INVENTION

The present invention relates to the field of food fermentation procedures involving lactic acid bacteria and in particular to improving the quality of dairy products like acidity, post-fermentation acidification, aroma, flavour, mildness, consistency and texture. Specifically, the present invention provides a method for making dairy products using the thermophilic *Streptococcus thermophilus* and the mesophilic *Lactococcus lactis* expressing small heat shock protein which allows fermentation to be carried out at temperatures higher than the regular fermentation temperatures. The thermophilic and mesophilic lactic acid bacterial species expressing said heat shock proteins also exhibit increased tolerance to lower pH and higher salt concentrations as well as reduced sensitivity to bacteriophage attack at the elevated fermentation temperatures.

### INTRODUCTION

In the food industry, the homofermentative, thermophilic lactic acid bacterium *Streptococcus thermophilus* is used as a starter species in dairy fermentations mostly in combination with thermophilic lactobacilli. Commercially, the most important of such fermentations is yoghurt production. Yoghurt results from the protosymbiotic growth of the two lactic acid bacteria *Streptococcus thermophilus* and *Lactobacillus delbrueckii* subsp. *bulgaricus.* Protosymbiosis characterises the metabolic interrelationships between organisms which in this respect means that both lactic acid bacteria promote each other's growth by providing nutrients required for optimal growth. As a consequence, acidification is rapid and results in final pH values as low as 3.5.

Although it is generally accepted that *Lactobacillus delbrueckii* subsp. *bulgaricus* is the yoghurt starter component mostly responsible for flavour development, results obtained from fermentation experiments using different combinations of *Streptococcus thermophilus* and *Lactobacillus delbrueckii* subsp. *bulgaricus* strains indicate a considerable impact of *Streptococcus thermophilus* on flavour development (C. Möller, W.Bockelmann, and K. J. Heller, unpublished).

The number of consumers preferring yoghurt with milder aroma has been increasing during recent years. Such milder taste may be achieved if fermentation stops already at pH values between 4.0 and 4.7. In addition, post-fermentation acidification is a major cause of bitterness and/or sourness in yoghurt. In order to overcome this undesirable characteristic of yoghurt, the development of bitterness and/or sourness can be inhibited by using starter combinations showing no or only little post-fermentation acidification.

In addition to yoghurt, thermophilic starter cultures are also used for production of different types of hard cheese, as well as semi-hard cheese like mozzarella, and acid curd cheese. Especially for the latter cheese the final pH obtained after fermentation has a considerable impact on the quality of the product.

The mesophilic lactic acid bacterium *Lactococcus lactis* is used as a starter component for many cheese varieties and butter milk. While with cheese production usually acidification is terminated well above pH 5.0 by cutting the curd and concomitant removal of the fermentable lactose, butter milk fermentation is terminated at a pH of 4.6, which is very close to the lowest pH reached by this organism.

In warm countries, like e. g. Egypt, keeping-time of raw milk is very short, due to unsatisfactory cooling systems. To overcome the problem of rapid spoilage, NaCl is added to the milk in relatively high quantities. This, on the other hand, interferes with subsequent dairy fermentations. Therefore, there is a strong demand for salt-tolerant starter cultures in such countries.

Dairy fermentations are carried out by starter organisms with different temperature requirements. Fermentations involving starter organisms with different temperature requirements at one temperature lead to sub optimal growth of one of the organisms. By way of example, in the case of yoghurt-production, cell count numbers *of Lactobacillus delbruekii* ssp. *bulgaricus* in the final product generally are too low due to fermentation at sub optimal temperatures.

Thus there is a pressing need to develop lactic acid bacteria which can ferment milk at higher temperatures and salt concentrations than their regular fermentation temperatures and salt concentrations and species which remain viable for longer at lower pH values. On the other hand, fermentation at higher temperature would suppress the raise of contaminants. For this reason the salt concentration might be reduced with benefit for human health.

Koch et al (1998; Journal of Bacteriology 180: 3873-3881) teaches two temperature-sensitive dnaK *Lactococcus lactis* mutants with C-terminal deletions in dnaK.

Morel et al (2001; Letters in Applied Biology 33: 126-130) teaches a recombinant *E*. *coli* which expresses the orfB, orfC and hsp 18 genes from *Oenococcus oeni -* and that the expression of these genes results in an improved viability of the host strain at pH 4.2.

De Angelis et al (2001; Microbiology 147: 1863-1873) teaches the *Lactobacillus sanfranciscensis* strain CB-1, a sourdough lactic acid bacterium, can withstand low pH after initial exposure to sub-lethal acid conditions. In addition, De Angelis et al teach two *Lactobacillus sanfranciscensis* mutants (CB1-5R and CB1-7R) which are constitutively acid-tolerant.

US 6,153,404 teaches a recombinant nucleotide sequence which comprises a regulatory sequence for initiating transcription (wherein said regulatory sequence includes a promoter having a sequence in association with a motif GCACTC 9N GAGTC) and a sequence coding for a heterologous polypeptide which can be expressed under control of said promoter. US 6,153,404 teaches that preferably the promoter is a promoter associated with a heat shock protein.

SU 739100 discloses a salt tolerant bacterial strain used for making cheese.

Aspects of the present invention are presented in the accompanying claims and in the following description and drawings. These aspects are presented under separate section headings. However, it is to be understood that the teachings under each section are not necessarily limited to that particular section heading.

### SUMMARY OF THE INVENTION.

Intriguingly, the inventors of the present patent application show that certain species of lactic acid bacteria which express small heat shock proteins can ferment milk at temperatures and/or salt concentrations higher than the normal milk fermentation temperatures and/or salt concentrations. The inventors of the present patent application further show that lactic acid bacteria which express heat shock proteins remain viable for longer at lower pH conditions and due to the high fermentation temperature become less sensitive to bacteriophage attack.

Importantly, the inventors have found that the lactic acid bacteria of the present invention when incubated with milk can produce a fermentation product with milder taste characteristics when compared to the fermentation product produced by corresponding lactic acid bacteria which do not express heat shock proteins.

Accordingly, the invention pertains in a first aspect to a method of preparing mild fermentation based dairy products, said method comprising the steps of : combining milk with a lactic acid bacterium capable of expressing a small heat shock protein, and culturing the milk/bacterium mixture at a temperature higher than a regular fermentation temperature and/or at a salt concentration higher than a regular fermentation salt concentration wherein the fermentation product has milder taste characteristics when compared to the fermentation product produced by the corresponding lactic acid bacteria which do not express small heat shock proteins.

In another aspect, there are provided thermophilic and/or mesophilic lactic acid bacteria which are used in dairy fermentation processes, which carry genes coding for small heat shock proteins and which produce a fermentation product with milder taste characteristics when compared to the fermentation product produced by the corresponding thermophilic and/or mesophilic lactic acid bacteria which do not express the small heat shock proteins of the present invention.

Preferably, the pH of the product is above 4.0.

In another aspect the present invention provides a method comprising combining milk with a first and a second lactic acid bacterium, in which each at least one of the first and/or second lactic acid bacteria is capable of expressing a small heat shock protein, and culturing the milk/bacterium mixture as specified.

In another aspect, the present invention also provides a method in which each of the first lactic acid bacterium and the second lactic acid bacterium comprises a mesophilic bacterium.

In another aspect, the present invention also provides a method in which each of the first lactic acid bacterium and the second lactic acid bacterium comprises a thermophilic bacterium.

In another aspect, the present invention also provides a method in which the milk is combined with a starter culture comprising a first thermophilic lactic acid bacterium prior to the addition of a second thermophilic lactic acid bacterium.

In another aspect, the present invention also provides a method in which the first lactic acid bacterium comprises a mesophilic lactic acid bacterium and the second lactic acid bacterium comprises a thermophilic lactic acid bacterium.

In another aspect, the present invention also provides a method in which the milk is combined with a starter culture comprising a mesophilic lactic acid bacterium prior to the addition of a thermophilic lactic acid bacterium.

In another aspect, the present invention also provides a method where each mesophilic bacterium is independently selected from a group consisting of but not limited to: *Lactococcus* spp, *Leuconostoc* spp, *Lactococcus lactis, Lactococcus lactis* subspecies *cremoris,* and *Lactacoccus lactis* subspecies *lactis.*

In another aspect, the present invention also provides a method where each thermophilic bacterium is independently selected from a group consisting of but not limited to: *Streptococcus* spp, *Bifidobacterium* spp, *Pediococcus* spp, *Lactobacillus* spp, *Streptococcus thermophilus, Lactobacillus delbrueckii* or *Lactobacillus delbrueckii* subspecies *bulgaricus.*

In yet another aspect, there are provided mesophilic and thermophilic lactic acid bacteria which have increased acid tolerance.

In a further aspect, there are provided mesophilic and thermophilic lactic acid bacterial species which have decreased sensitivity to bacteriophage attack as a result of the elevated fermentation temperature.

In yet a further aspect, the invention relates to milk that is obtained by the method of the present invention and a fermentation product with milder taste characteristics that is obtained by fermenting such a milk, such as yoghurt, and acid curd cheese, and hard cheese, and semi-hard cheese like mozzarella, and fresh cheese, and quark, and butter milk, and cottage cheese, when compared to the fermentation product produced by the corresponding lactic acid bacteria which do not express the small heat shock proteins of the present invention.

In yet another aspect, the invention relates to milk that is obtained by the method of the present invention and a fermentation product that is obtained by fermenting such as milk wherein the fermentation product can be selected from a group consisting of acid curd cheese, hard cheese, semi-hard cheese like mozzarella, fresh cheese, quark, butter milk, Swiss type cheese or cottage cheese.

In yet a further aspect, the invention relates to milk that is obtained by the method of the present invention and milder taste characteristics yoghurt that is obtained by fermenting such a milk when compared to the yoghurt produced by the corresponding lactic acid bacteria which do not express the small heat shock proteins of the present invention.

In another aspect, the present invention provides a method of fermenting milk in which the lactic acid bacterium comprises a recombinant lactic acid bacterium, which has been engineered to express a small heat shock protein at a higher level than a corresponding unmodified bacterium.

In another aspect, the present invention provides a method of fermenting milk in which the lactic acid bacterium comprises a recombinant lactic acid bacterium, which has been engineered to over-express a small heat shock protein when compared to a corresponding unmodified bacterium.

In another aspect, there are provided thermophilic and/or mesophilic lactic acid bacteria which are used in dairy fermentation processes, which carry genes for small heat shock proteins.

It is known that small heat shock protein(s) (sHsp) form one of the families of molecular chaperones preventing the irreversible aggregation and assisting in the refolding of denatured proteins. These proteins exist in all organisms and are characterized by a low molecular mass in the range of 12-30 kDa, oligomeric structure and the presence of a conserved domain. This domain is highly similar to a-cristallins of vertebrates. It is preferred that the small heat shock proteins which are capable of being used in the methods of the present invention have a molecular mass in the range of 16-18 kDa.

Typically the term "small heat shock protein(s) or (sHsp) " refers to an ATP- independent sHsp capable of binding and stabilizing denatured proteins and preventing their irreversible aggregation. Proteins stabilized in this way can be subsequently refolded by an ATP-dependent chaperone system.

Analysis of complete bacterial genome sequences revealed, that most bacteria have only a small number of small heat shock proteins. In the lactic acid bacteria only a few strains of *S*. *thermophilus* carry genes for sHsps, which are located on small plasmids separate from the bacterial genome.

### DETAILED DESCRIPTION OF THE PRESENT INVENTION.

### Improved lactic acid fermentation.

A primary objective of the present invention is to provide a method to improve the properties of fermentation based milk products. This is, in respect of yoghurt, achieved by providing a method for preparing yoghurt which has highly desirable characteristics with respect to milder aroma, acidity, post-fermentation acidification, taste and appearance and which additionally has an extended shelf life as reflected in the reduced whey production, the mildness of the aroma and higher density of live cultures i. e. the staling of the fermented milk product is retarded relative to a milk product made without use of the microorganisms of the present invention.

As used herein, the term "milder taste characteristics" refers to fermented milk products characterised with significantly reduced bitterness and/or sourness. In addition or in the alternative, the term "milder taste characteristics" may also be interpreted with respect to the pH of the product. Accordingly, the product of the present invention which has a mild taste characteristics has a pH above 4.0, preferably in the range 4.0 to 5.0, even more preferably 4.0 to 4.7. Accordingly, milder taste characteristics fermentation milk products refer to the fermentation milk products obtained by incubating the lactic acid bacteria of the present invention with milk according to the method of the present invention.

As used herein the term "corresponding lactic acid bacteria which do not express the small heat shock proteins of the present invention" refers to lactic acid bacteria which have been cured of (i.e. they have lost) the plasmid which carries the small heat shock proteins of the present invention (see Examples). The curing of a bacteria from a plasmid can be achieved by methods known in the art. Alternatively, the lactic acid bacteria which do not express the heat shock proteins of the present invention may still contain the plasmid but the genes coding for the shsp may be specifically modified using genetic recombination and/or mutagenesis techniques to prevent the expression of the genes.

Although it is presently preferred to use the method of the invention for yoghurt production, the use of the method for any other type of fermentation based milk product such as hard cheese, semi-hard cheese like mozzarella, acid curd cheese, quark, butter milk, fresh cheese and cottage cheese the quality of which can be improved by the addition of the microorganisms of the present invention, is also contemplated.

The milk in respect of the present invention can be obtained from a ruminant animal, preferably selected from a group consisting of buffalo, cow, sheep, lama, goat or camel. The milk can also be skimmed or semi-skimmed. The milk may also be derived from plants such as soy or rice or it can be synthetically generated milk.

Synthetically generated milk is a term used in the art to describe an artificial product which is similar to milk. The composition of the synthetic milk may be specifically designed to exclude for example certain components to which consumers may be intolerant or allergic. The synthetic milk can also be a fortified milk. By way of example, the fortified milk may contain additional compounds which may or may not have nutritional value such as minerals, vitamins, polysaccharides, oligosaccharides, thickeners or water absorbing materials.

Generally, temperature range for growth of an organism is a characteristic of considerable taxonomical value. Depending on their preferred growth temperature, bacteria in general and that includes lactic acid bacteria are divided broadly into mesophilic and thermophilic bacteria. It is known that mesophilic bacteria have an optimum temperature of between 20°C to 44 °C. While the optimum temperature of thermophilic bacteria is in the range of between 35°C to 55 °C.

The present invention relates to methods for producing different milk products by fermenting milk with combinations of mesophilic and/or thermophilic lactic acid bacteria.

According to the present invention, mesophilic lactic acid bacteria are defined as bacteria that have a regular growth temperature in the range of between 20-30 °C.

However, mesophilic lactic acid bacteria which exhibit a regular growth temperature of between 30°C and 44 °C may also be used in the methods of the present invention.

According to the present invention, thermophilic lactic acid bacteria are defined as bacteria that have a regular growth temperature in the range of between 35°C - 47 °C.

However, thermophilic lactic acid bacteria which exhibit a regular growth temperature of between 50°C to 55 °C may also be used in the methods of the present invention.

The summary below lists a number of different mesophilic and thermophilic lactic acid bacteria which is for the purposes of illustration and not in any way limited to the specific lactic acid bacteria included in the summary below.

### Optimal growth temperature of starter cultures

| Classification of culture | Species | Range of optimal growth temperature |
|---|---|---|
| mesophilic culture | *Lactococcus lactis* | 18 - 30°C |
| | Leuconostoc | 18-30°C |
| thermophilic culture | *Streptococcus thermophilus* | 35 - 42 °C |
| | Lactobacillus del. *bulgaricus* | 43 - 46°C |

The present method comprises as an essential step that an effective amount of lactic acid bacteria of the invention which under fermentation conditions are capable of fermenting milk at a higher temperature than regular fermentation temperatures: up to 43 °C for the mesophilic lactococci (especially for *Lactococcus lactis* subsp. *cremoris*) and up to 55°C for the thermophilic *Streptococcus thermophilus.*

The fermentation temperature of yoghourt is 42 °C (or between 40-43 °C), which is a compromise between the optima of the two starter organisms *Streptococcus thermophilus* (39 °C) and *Lactobacillus delbrueckii* subsp. *Bulgaricus* (45°C). When the *Streptococcus thermophilus* carries the shsp the optimum fermentation temperature is altered and the optimum temperature of the organism is increased. This means that you can ferment the yoghourt at 45 °C and then both *Lactobacillus delbrueckii* subsp. *bulgaricus* and *Streptococcus thermophilus* carrying shsp will grow at optimum temperatures.

Accordingly, mesophilic lactic acid bacteria expressing small heat shock proteins are capable of fermenting milk at temperatures which are higher than the corresponding mesophilic lactic acid bacteria which do not express small heat shock proteins.

The thermophilic lactic acid bacteria *Streptococcus thermophilus* when expressing the small heat shock proteins is capable of fermenting milk in the range of 42 °C to 55 °C which is higher than the optimum temperature for the corresponding *Streptococcus thermophilus* which do not express small heat shock proteins.

Importantly, an effective amount of small heat shock protein expressing *Streptococcus thermophilus* bacteria when incubated with an effective amount of *Lactobacillus del. bulgaricus* can ferment milk at higher than normal temperatures, which would lead to the production of a milder taste characteristics product.

As used herein, the term "effective amount" describes an amount of thermophilic and/or mesophilic bacterial species such as *Streptococcus thermophilus* or *Lactococcus lactis* respectively which is sufficient to ferment detectable amount of sugar compounds present in the milk. More specifically, presence of the microorganisms of the present invention may not only result in detectable fermentation of sugar compounds in milk but which in addition results in the formation of fermentation end products at a level which result in improved properties of the milk products, such as significantly improved acidity, texture, taste, appearance and the mildness of the aroma which can be ascribed to the addition of the microorganisms of the present invention.

In one aspect, *Streptococcus thermophilus* which ferments milk at elevated temperatures is used for the production of mild aroma fermented milk products which comprise but are not limited to yoghurt, acid curd cheese, hard cheese, semi- hard cheese like mozzarella, or fresh cheese.

In another aspect, *Lactococcus lactis* which ferments milk at elevated temperatures is used for the production of mild aroma fermented milk products which comprise but are not limited to quark, butter milk, fresh cheese, or cottage cheese.

As used herein the terms "elevated temperatures" or "higher temperatures" are used interchangeably and refer to the ability of mesophilic lactic acid bacteria and thermophilic lactic acid bacteria to ferment milk at higher than normal fermentation temperatures.

For mesophilic lactic acid bacteria the term "elevated temperatures" refer to the fermentation of milk at between 39°C - 48 °C, preferably between 41°C - 47 °C, more preferably between 43°C - 46 °C, even more preferably between 43°C - 46 °C and most preferably at 43 °C compared to the regular fermentation temperature range (see Summary above) In the case of thermophilic lactic acid bacteria the term "elevated temperatures" refers to the fermentation of milk at between 48 °C - 58°C preferably between 53 °C - 57°C, more preferably between 54 °C - 57 °C, even more preferably between 54 °C - 56 °C and most preferably at 55 °C compared to the regular fermentation temperature range (see Summary above).

In addition, the availability of mesophilic starter bacteria growing at temperatures above 39 °C allows new combinations of mesophilic and/or thermophilic starters for production of new fermented products.

In yet another aspect, lactic acid bacteria with reduced bacteriophage attack sensitivity are used. This is as a result of the ability of the lactic acid bacteria according to the present invention to ferment milk at elevated temperatures.

In another aspect, lactic acid bacteria with increased tolerance to higher concentrations of salt for example higher than 1 %, preferably higher than 2 %, and more preferably higher than 2.5 % are used. It is of note that the normal salt concentration of milk is in the range of 0.7 to 0.8 %.

This feature of the microorganisms are particularly important for southern countries where salt is added to milk to inhibit growth of spoilage and pathogenic microorganisms. Such milk may be more easily fermented by the lactic acid bacteria when producing milder taste characteristics milk product obtained by fermenting such a milk when compared to the milk product produced by the corresponding lactic acid bacteria which do not express the small heat shock proteins.

In another aspect, the present invention provides a fermentation product comprising lactic acid bacteria expressing small heat shock proteins with increased shelf life of the product when stored at 4 °C

In another aspect, yoghurt with thermophilic starter cultures above 42°C with higher cell count numbers of viable *Lactobacillus sp.* in the final product is provided.

In another aspect, the present invention provides a method of fermenting milk in which the lactic acid bacterium comprises a recombinant lactic acid bacterium, which has been engineered to express a small heat shock protein at a higher level than a corresponding unmodified bacterium which can carry out milk fermentation at temperatures and/or salt concentrations higher than regular fermentation and/or salt concentrations.

In another aspect, the present invention provides a method of fermenting milk in which the lactic acid bacterium comprises a recombinant lactic acid bacterium, which has been engineered to over-express a small heat shock protein when compared to the corresponding unmodified bacterium which can carry out milk fermentation at temperatures and/or salt concentrations higher than regular fermentation and/or salt concentrations.

### EXAMPLES.

The present invention will now be illustrated by way of examples where reference is made to the following figures and tables.
Fig. 1A shows an SDS-PAGE.
Fig. 1B shows a Western Blot.
Fig. 2A and B show graphs.

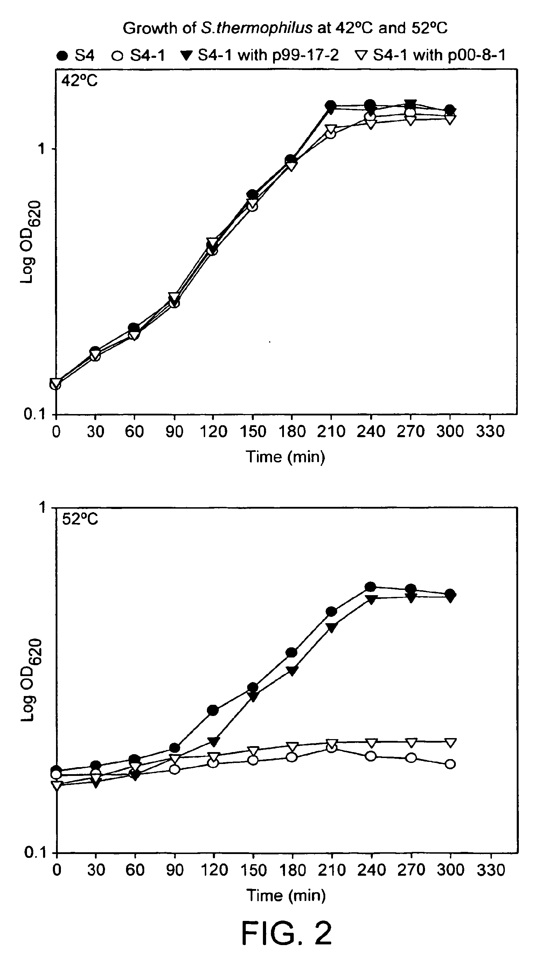

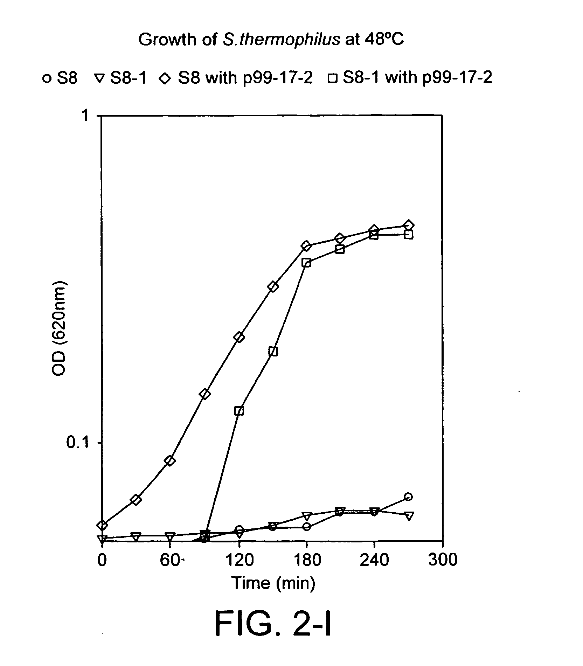

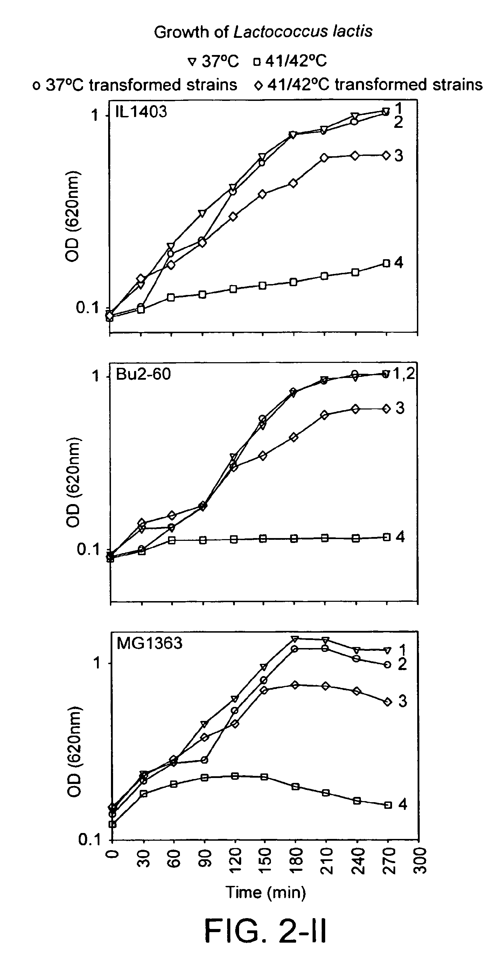

Fig. 2-I shows a graphic representation of log OD 620.
Fig. 2-2 shows a graph.

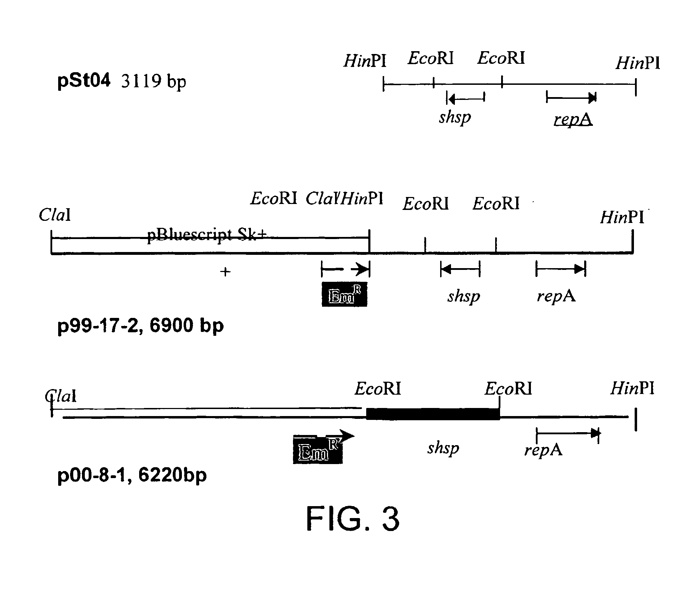

Fig. 3 shows a schematic.

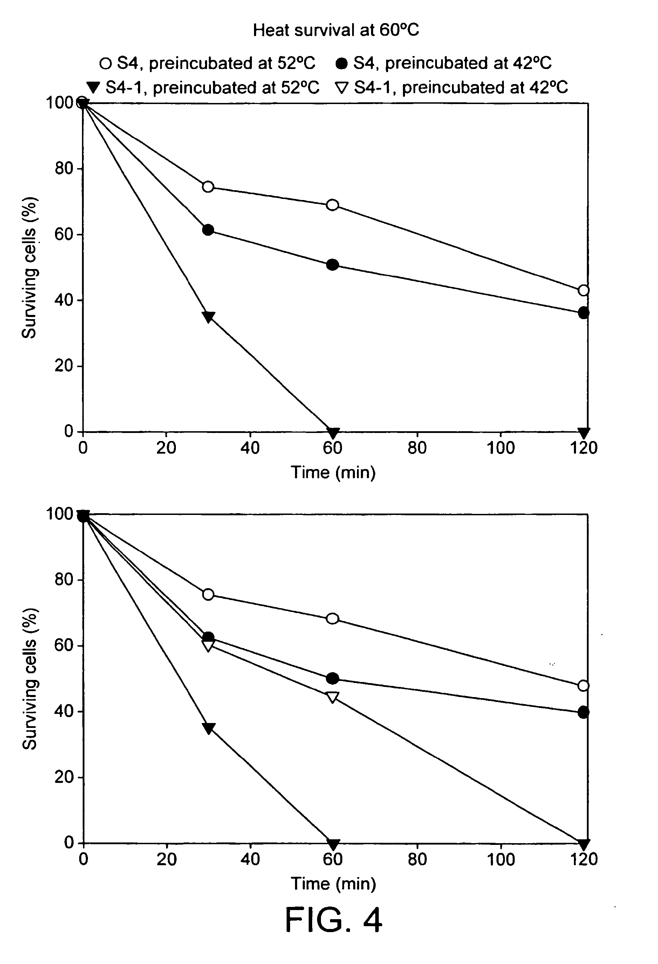

Fig. 4 shows a graphic representation of % surviving cells.

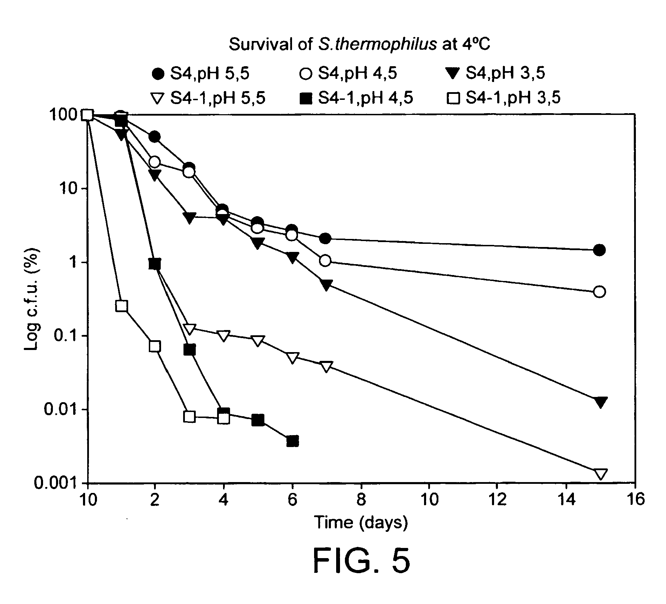

Fig. 5 shows a graphic representation of log colony forming units (%).

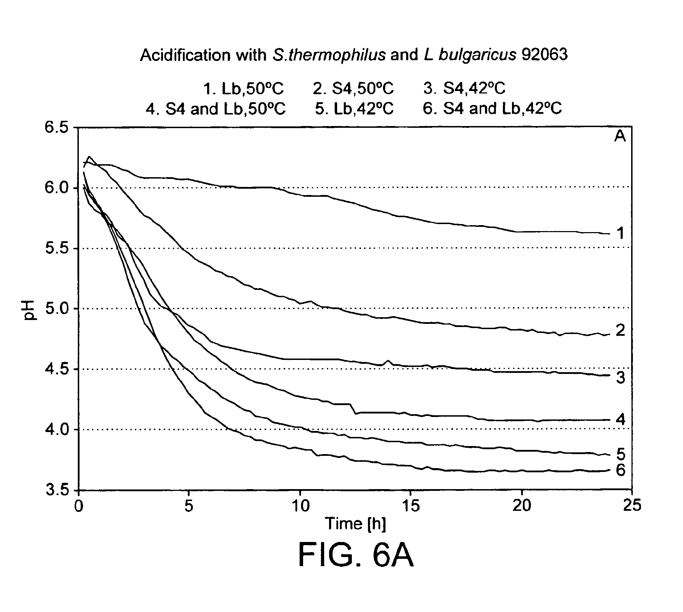

Fig. 6A shows a graph.

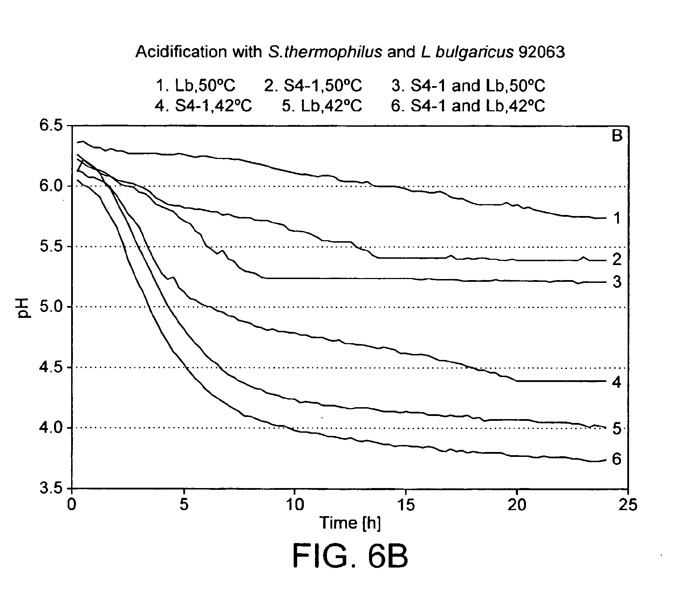

Fig. 6B shows a graph.

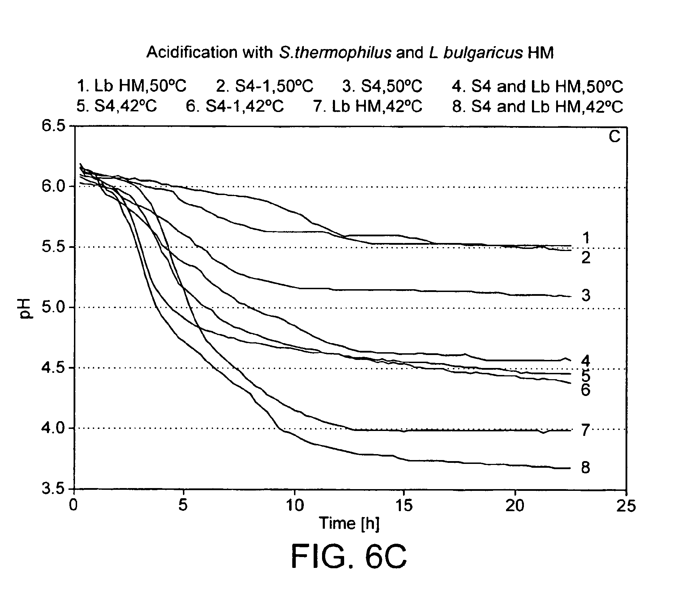

Fig. 6C shows a graph.

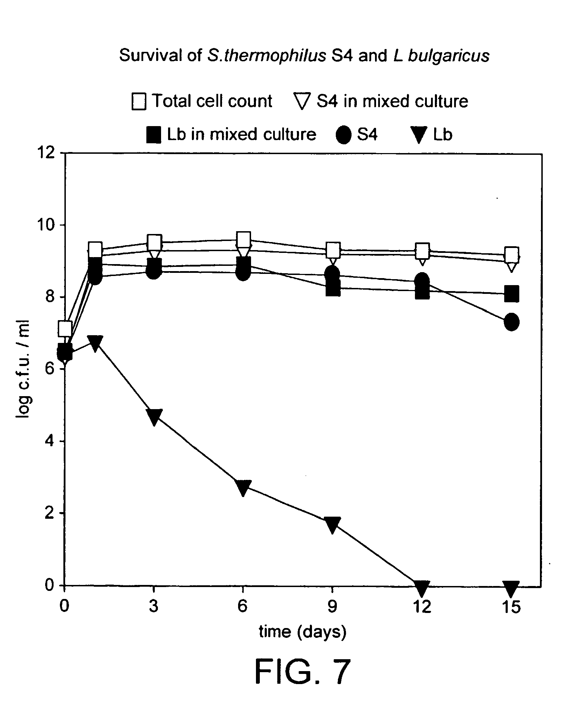

Fig. 7 shows a graphic representation of log colony forming units/ml.

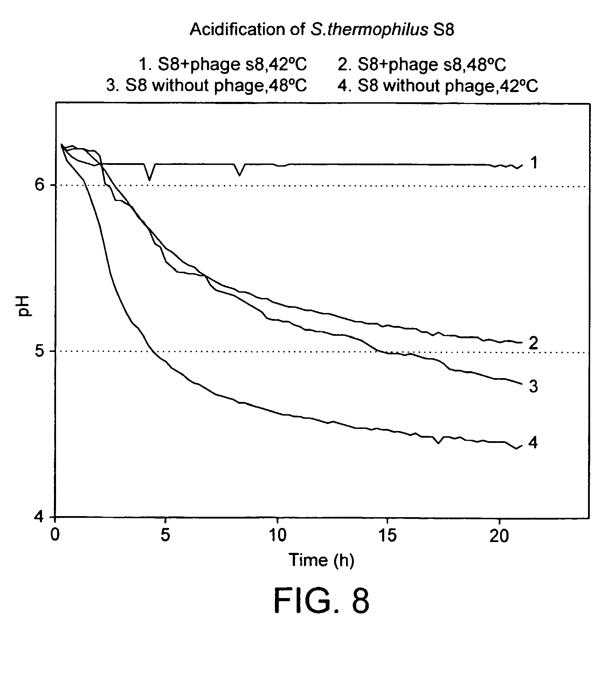

Fig. 8 shows a graph.

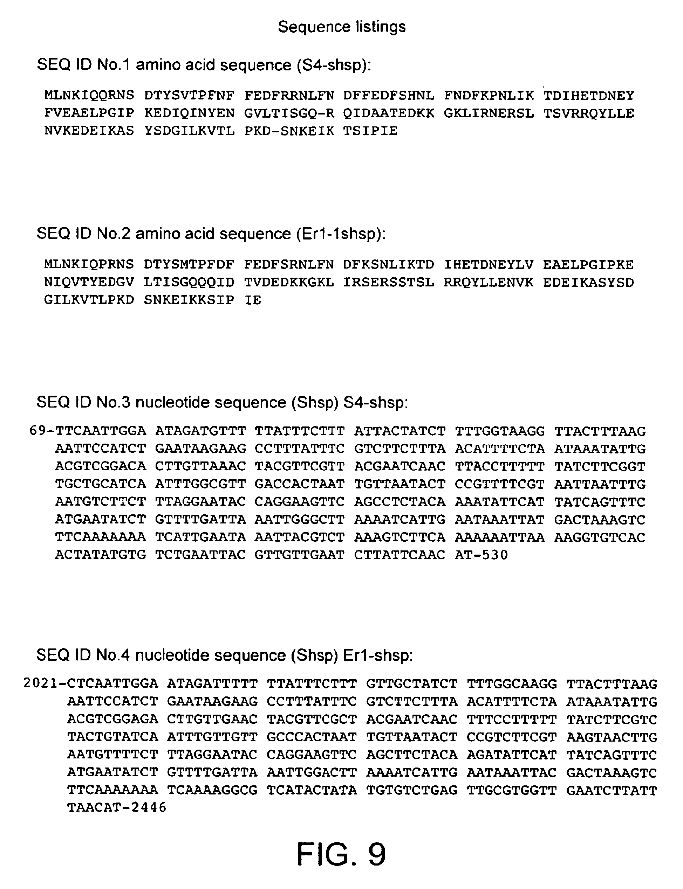

Fig. 9 shows sequence listings.

Table 1. Bacterial strains and bacteriophage.
Table 2. Change of the pH-values of yoghurt produced with S. thermophilus S4 and *Lactobacillus bulgaricus* 92063 at 42°C and 50 °C during storage at 4 °C.

### The Figures explained in greater detail.

### Figure1. A.

SDS-PAGE of the soluble proteins of S. thermophilus S4-1 (lane 2) and S4 w/o and with heat shock after incubation for 60 min at 40°C (lanes 3 and 4), 45 °C (lanes 5 and 6) and55 °C (lanes 7 and 8). Lane 1 contains protein size markers with molecular masses of 6.5 ; 14.2 ; 18.4 ; 29; 48.5 ; and 66 kDa. Lane 9 contains partially purified sHsp isolated from an overexpressing *E*. *coli* strain. The heat shock was performed at52 °C for 30 min prior to incubation at the indicated times.

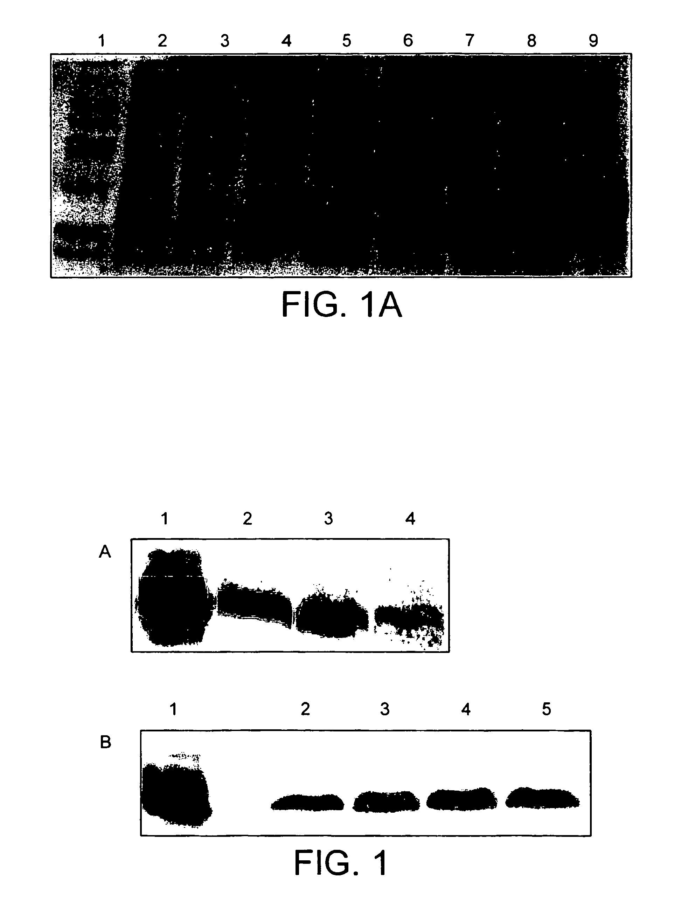

Figure1. B. Induction of sHsp expression in *S*. *thermophilus* S4. Cells of S. thermophilus S4 and S4-1 grown inLTM17 medium to the late log-phase of growth(OD620-1. 0) were collected, washed with sugar-free medium, and resuspended in TM17 containing 0.05 % glucose.5ml aliquots were incubated for 30 min at 37, 46, and 52 °C (A) or at 52 °C for 5 - 60 min (B). Cell extracts were prepared and Western blot analysis was performed. A: partially purified sHsp from E. coli (positive control), lane 1; S thermophilus S4 incubated at 52, 46, and 37 °C, lanes 2 to 4; S4-1 incubated at 52 °C (negative control), lane 5. B: positive control, lane 1; 5 min, lane 2; 15 min, lane 3; 30 min, lane 4; 60 min, lane 5.

Figure 2. A. Growth curves showing *S*. *thermophilus* S4 (filled circles, (● S4) ), its plasmid-cured derivative S4-1 (open circles, (○ S4-1) ), S4-1 transformed with the recombinant plasmids p99-17-2(shsp+) (filled triangles,(▼ S4-1) ), and p00-8-1 (shsp') (open triangles,(∇ S4-1)) incubated at42 C.

Figure 2. B. Growth curves showing *S*. *thermophilus* S4 (filled circles,(● S4)), its plasmid-cured derivative S4-1 (open circles, (○ S4-1)), S4-1 transformed with the recombinant plasmids p99-17-2 (shsp+) (filled triangles,(▼ S4-1)), and p00-8-1(shsp') (open triangles,(∇ S4-1)) incubated at 52 °C.

Figure 2-1: Growth at 48°C *of Streptococcus thermophilus* S8 (circles, (○ S8) ), its plasmid-free derivative S8-1 (triangles,(∇ S8-1)) and of strains S8 and S8-1 transformed with the recombinant plasmid p99-17-2 (diamonds, squares, (0 S8 with p99-17-2, □S8-1with p99-17-2).

Figure 2-2: Growth of *Lactococcus lactis* strains Bu2-60, IL 1403, and MG1363 and its derivatives transformed with the recombinant (shsp+) plasmid p99-17-2. Bu2-60 IL1403, MG1363 at37 or 41/42 °C (1-triangles and 4-squares, (∇ 37°C and □ 41/42 °C)) ; transformed strains at 37 or 41/42 °C (2-circles and 3-diamonds, (○ 37 °C and 0 41/42 C)).

It is seen from the figures that all the *Lactococcus lactis* strains grow well at 37°C (1) but not at 42 °C (4). The strains transformed with the plasmid p00-17-2 increases growth at 42°C (3) whereas growth at 37 °C does not change.

Figure 3. Physical maps of the plasmids pSt04, p99-17-2, and p00-8-1.

Figure 4. Heat survival curves of S. thermophilus S4 (circles, (○) S4, preincubated at 52 °C and (●) S4, preincubated at 42 °C)) and its plasmid-free derivative S4-1 (triangles, (∇) S4-1, preincubated at 52 °C and (▼) S4-1, preincubated at 42°C) in TM17 medium at 60 °C. Prior to exposure to 60 °C, cells were preincubated at 42 °C (filled symbols, (●) S4 and (▼) S4-1) ) or52 °C (open symbols, (○) S4 and (∇) S4-1)) for 30 min.

It is seen from Figure 4 that S4 shows better survival than the plasmid free strains S4- 1.

Figure 5. Survival of S. thermophilus S4 and its plasmid-free derivative S-1 during storage at 4°C at different pH-values. Cells were resuspended in growth medium preadjusted to the appropriate pH by addition of lactic acid and stored at 4 °C up to 15 days. The cell count was determined every third day by plating proper dilutions on LTM17 agar. (●) S4, pH 5,5 ; (○) S4, pH 4,5 ;(▼) S4, pH 3,5 ;(∇) S4-1, pH 5,5 ;(■) S4-1, pH 4,5 ;(□) S4-1, pH 3,5.

From the Figure it can be seen that S4 exhibits better survival at all the mentioned pH values. The survival of S4-1 is dramatically reduced after 1-2 days storage and after 4-6 days almost no bacteria have survived.

Figure 6. Skim milk acidification in single and mixed strain fermentations with S. *thermophilus* S4 and S4-1 and *L. bulgaricus* 92063 at different temperatures. Skim milk was inocculated with 5 x 10⁶ CFU/ml for each strain in the mixed strain and10⁷ CFU/ml for the single strain fermentations. The decrease of the pH was followed automatically with a multichannel pH-meter.

Figure 6. A: Lb 92063 at 50 °C (1); S4 at50 °C (2); S4 at 42°C (3); S4 and Lb at 50°C (4); Lb at 42°C (5); S4 and Lb at 42°C (6). following:

Figure 6. B: Lb 92063 at 50 °C (1); S4-1 at 50°C (2); S4-1 and Lb at 50°C (3); S4-1 at 42°C (4); Lb at 42°C (5); S4-1 and Lb at 42 °C (6).

Figure 6. C: Lb HM at 50 °C (1); S4-1 at 50°C (2); S4 at 50°C (3); S4 and Lb HM at 50°C (4); S4 at 42°C (5); S4-1 at 42°C (6); Lb HM at 42°C (7); S4 and Lb HM at 42 °C (8).

From figure 6A and B it is seen that curve no 6 in both figures corresponds to a normal yoghourt fermentation. After 15-20 hours fermentation a pH of around 3.6 is reached. Curve 4 in figure 6A and 6C shows the fermentation carried out at 50 °C where the *Streptococcus thermophilus* carries the shsp. These fermentations reach a pH level around 4.0-4. 1. Curve 3 in figure 6B shows that fermentation with S4-1 and Lb 92063 ends at a pH around 5.2.

Figure 7. Survival of *S*. *thermophilus* S4, and *L. bulgaricus* after growth in single or mixed strain culture at 50°C and subsequent storage. Skim milk was inoculated to final cell density of 10⁷ CFU/ml and incubated for 24 h at 50 °C. Subsequently the fermented milk was stored at4 °C for 15 days. The total cell count for each strain as well as the total cell count (counts of *Lactobacillus* and S4 together, was determined every third day. Total count (open squares, (□)) ; S4 and Lb in mixed culture (open triangles (∇ S4) and filled squares(■ Lb) ); S4 (filled circles,(●) S4); Lb (filled triangles,(▼) Lb).

Figure 8. Acidification of *S*. *thermophilus* S8 and its transformed derivative S8 (pSt04-shsp) at42 and 48 C. Skim milk was inoculated with 10⁷ CFU/ml and incubated at the indicated temperature in the absence or presence of bacteriophage s8 at a multiplicity of infection (m.o.i.) of 10. Acidification was followed automatically with a multichannel pH-meter. S8(pSt04) + phage s8, 42°C (1); S8(pSt04) + phage s8, 48 °C (2); S8(pSt04) w/o phage, 48 °C (3); S8(pSt04) w/o phage, 42 °C (4).

Figure 9. Sequence listings.
SEQ ID No : 1
SEQ ID No : 2
SEQ ID No: 3
SEQ ID No: 4
Specific Examples.

General features of plasmidspSt04 and pER1-1 carrying genes for small heat shock proteins.

The nucleotide sequence of plasmids pSt04 and pER1-1, which belong to DNA- homology group I of plasmids from *Streptococcus thermophilus* (Janzen et al. 1992) were determined (Accession numbers: AJ 242477 and AJ 242476). These plasmids share a DNA region of about 1200 bp with homologies of more than 90% which is necessary for plasmid replication.

In addition to the repA gene, plasmids pSt04 andpER1-1 each possess a second ORF encoding a polypeptide of 155 and 142 amino acids (aa) residues (SEQ ID No:1 and SEQ ID No: 2) respectively, corresponding to molecular masses of 18,042 and 16,422 Da. These ORFs are positioned counter clockwise to the repA-genes. Putative ribosomal binding sites(GAa/gGAAAG) 8bp upstream of the start-codons arepreceeded by5'-TTGAAA... (16bp)... TATAAT promoter regions. The predicted gene products are highly similar (> 90%) to small heat shock proteins (sHsp) observed in other *S*. *thermophilus* strains (O'Sullivan et al. 1999, Somkuti et al. , 1998). These sHsps belong to a family of shock response proteins common in prokaryotic and eukaryotic organisms. The sHsp synthesis significantly increases from a low basic level at pH-values below pH 4.5 or at temperatures above 45°C and could be further increased by a short, 30 min shock at pH 4.0 or at 52°C (Figure 1).

### Functions of small heat shock proteins.

### i) Growth at elevated temperatures and thermoresistance

The growth behaviour at different temperatures of S. thermophilus strains S4 andER1, carrying plasmidspSt04 and pER1-1, respectively, was compared to that of the plasmid-cured derivatives S4-1 andER1-1 of these strains. At42 °C all strains grew with identical rates to final cell densities of about 2 x 10⁹ CFU/ml. At 45°C the plasmid- free strains grew somewhat slower but to almost the same final cell densities. At 52 °C only the plasmid (shsp)-bearing strains were able to grow with reduced growth rates to final cell densities of about 1 x 10⁹ (Figure 2).

To verify that the shsp-gene was responsible for the ability to grow at high temperature, plasmid-free strain S4-1 was transformed with recombinant plasmids p99-17-2 and p00-8-1. Plasmid p99-17-2 contains the entirepSt04 sequence cloned into the *E. coli* vector pBluescriptlI (SK+) and carries as selection marker for gram- positive bacteria the erythromycin-resistance gene from the *Staphylococcus aureus* plasmid pE 194. Plasmid p00-8-1 is a deletion derivative of p99-17-2 missing the entire shsp-sequence (Figure 3). Strains S4-1 (p99-17-2) andS4-1(p00-8-1) showed the same growth behaviour as S4 and S4-1, respectively, proving that the presence of the shsp- gene was responsible for the ability to grow at temperatures up to 52°C (Figure 2).

The recombinant plasmid p99-17-2 was subsequently transferred by electroporation into S. *thermophilus* strains S8 andS 11 as well as into the mesophilic *Lactococcus lactis* subsp. *lactis* strains IL1403 and Bu2-60 and *L. lactis* subsp. *cremoris* strain MG1363. The maximal growth temperatures for S8 and S11 were raised from 45°C up to 48 °C (Figure 2-1) and 52 °C (Figure 2), respectively and from 37 °C up to 42 °C for the lactococcal strains (Figure 2-2).

Compared to its plasmid-free derivatives *S*. *thermophilus* strains S4 and ER1 exhibited higher thermoresistance. About 50% of the plasmid-bearing but non of the plasmid- free cells survived incubation at 60 °C for 2 h. By a short heat-shock (30 min at 52°C) the thermoresistance could be further improved (Figure 4).

### ii) Acid tolerance

In addition to an increased maximal growth temperature and improved thermoresistance the presence of sHsp increased the acid tolerance. Cells expressing shsp showed a strongly improved viability upon storage at low pH. Even at pH 3.5, a pH value lower than in most fermented milk products, more than 40% of the cells survived storage for 15 days at 4°C, whereas no cells of the shsp-negative mutants were detectable after 7 days at identical conditions (Figure 5).

### iii) Salt tolerance.

*S. thermophilus* S4 and its plasmid-free derivative S4-1 were grown at sodium chloride concentrations from 0-3%. No obvious differences either in growth rate or the final cell densities were observed at NaCl-concentrations up to 1%. At salt concentrations from 1.5 to 2.5% the wildtype strain grew faster than the plasmid-cured strain. No growth occured at salt concentrations of 3% NaCl.

Application of thermoresistant *S*. *thermophilus* strains in mixed culture with *L. bulgaricus* for milk fermentation.

*S. thermophilus* strains carrying a plasmid-encoded shsp-gene show significantly increased thermo-and acid-resistence. To prove, whether the two available shsp- positive S. *thermophilus* strains, S4 and ER1, were suitable for yoghurt production, each of the strains was used in combination (1:1) with *Lactobacillus delbrueckii* subsp. *bulgaricus* (*L*. *bulgaricus*) 92063 and HM for skim milk fermentation. Mixed cultures of *L. bulgaricus* and isogenic derivatives of S4 and ER1, cured of the shsp-gene containing plasmids, served as a control.

Acidification during milk fermentation The strain combination S4/Lb92063 showed significant protosymbiotic effects in the 42°C as well as the 50 °C fermentations. At 42°C the pH dropped to values below pH 4.0 after 7 h and decreased to pH 3.6 after 24 h. At 50°C the pH-value was 4.3 after 8-10 h and 4.1 after 24 h (Figure 6A). The strain combination S4-1 with LB 92063 showed significant protosymbiotic effects at42 C, but no acidification was observed at 50 °C (Figure 6B).

With the strain combination, *S*. *thermophilus* ER1 and Lb 92063, similar results were obtained. The acidification activity was, however, slightly lower. The control fermentations (*S.thermophilus* ER1-2 and Lb 92063) at42 °C were very similar to the test-fermentation at this temperature; at 50°C acidification was unsufficient.

*S. thermophilus* S4 in combination with *L. bulgaricus* HM gave similar results as the combination S4/Lb 92063 (Figure 6C).

Cell counts during milk fermentation and subsequent storage

Starting with a cell number of about2x106 cfu/ml for each strain, the cell number increased in fermentations with the mixed culture S4/Lb92063 at50 °C to 10⁹ (S4) and 6 x 10⁸ (Lb) cfu/ml. In single-strain fermentations, strain S4 grew to cell numbers of about 5 x 10⁸ cfu/ml, whereas the plasmid-cured, shsp-negative strain S4-1 as well as the Lb 92063 grew only slowly to final cell densities of about8x106. After a 24h fermentation the fermented milk was stored at 4°C for 15 days. The cell numbers of the single strains as well as the total cell counts were determined every third day. At the end of the storage period only a slight decrease of the cell numbers was observed for S. thermophilus S4 and and Lb 92063. The high survival of Lb 92063 was particularly surprising, since this strain, grown in single strain culture at 50°C was no longer detectable after storage for 12 days (Figure 7). The reason for this positive effect on the survival of L. *bulgaricus* grown at 50°C in mixed culture together with a thermo resistant S. *thermophilus* is still unknown.

Production of yoghurt with a mixed-strain culture of *S*. *thermophilus* S4 and *L. bulgaricus* 92063 at 42 and 50 °C.

Pasteurised milk (3.5% fat) was inoculated with a mixed culture of S4 and Lb 92063 and incubated at 42 °C and 50 °C for 12 h. At the end of the fermentation the yoghurt products were tested for texture, pH, cell number, taste and aroma. The yoghurt products were subsequently stored at 4 °C for 2 weeks and tested every third day as above. Yoghurt produced at 42°C had a firm texture with slight whey separation, a pH- value of 3.6 to 3.5 and cell numbers of about 10⁹ cfu/ml for each of the strains. It tasted very acidic and had a sour aroma. Yoghurt produced at50 °C also showed a firm texture without whey separation, the pH was about 4.3 after fermentation and 4.0 at the end of the storage period (Table. 2). The cell numbers were comparable to the 42 °C-fermentation and decreased only slightly during storage. The yoghurt tasted mildly, comparable to a well-fermented "Joghurt-Mild" and had a rich aroma. Fermentation at50 °C with a proper combination of a thermo resistant S. *thermophilus* and a *L. bulgaricus* resulted in a yoghurt with the characteristic of a"Joghurt-Mild", but with the original microflora of a classical yoghurt and high cell density even after storage for at least the shelf live of the product.

Influence of the fermentation temperature on the bacteriophage sensitivity.

To test the effect of the fermentation temperature on the phage sensitivity, the acidification activity of S. thermophilus S8 transformed with the shps-plasmidpSt04 was determined at 42 and 48°C in the absence or presence of the virulent phage s8 at a multiplicity of infection (M. o. i.) of about 10. At42 °C no acidification was observed, whereas at 48°C a final pH of about 5.0 was obtained (Figure 8). This indicates, that even at this unrealistic high phage load higher fermentation temperatures have a protective effect against phage attack.

### Material and Methods

### Bacterial strains, bacteriophages and media.

The strains used in this study are listed in Table 1. S. thermophilus and *Lactococcus lactis* were grown in TM 17 medium (Krusch et al. 1987) supplemented with 1 % lactose, *L*. *bulgaricus* was grown in MRS medium (Merck, Darmstadt, Germany). *E. coli* was grown at 37°C in Luria broth (Sambrook et al. 1989). When appropriate, antibiotics were added as follows: 3 or 5 µg/ml erythromycin for *S*. *thermophilus* and *Lactococcus lactis,* respectively, and100g erythromycin per ml for *E*. *coli.* Blue-white selection was performed as described by Sambrook et al.,1989). Acidification was determined in 9 % skim milk medium, pH was monitored by an 8-channel pH- meter(Ingenieurbro Messelektronik, Chemnitz, Germany). Bacteriophage s8 was propagated as described by Sambrook et al.,1989) and added to the fermentation assays at a multiplicity of infection (m.o.i.) of about 10.

### DNA isolation and manipulation.

Plasmid DNA was isolated from *E*. *coli* with a NucleoSpin kit (Macherey-Nagel,Duren, Germany) and from *S*. *thermophilus* and *L. lactis* by adapting the method accordingly. Enzymes were purchased from New England Biolabs, Beverley, Mass. and used according the manufacturers instructions.

### Transformation.

E. coli was electrotransformed by a procedure recommended by Bio-Rad Laboratories, Richmond,Calif., *L. lactis* as described by Holo and Nes (1989) andS. thermophilus according to Mohamed (PhD-thesis, Univ. Kiel, Germany, 2002).

### Construction of plasmids p99-17-2 andp00-8-1.

PlasmidpSt04 was linearized by digestion with Hint 1 I and cloned into pBluescrips II SK+ (*Cla*I). The recombinant plasmid p99-16-5 was subsequently genetically marked by insertion of aerthromycin-resistance gene cassette into the BamHI site of pBluescript. A small EcoRI fragment was removed resulting inp00-8-1 which lacks the entire shsp-gene. The recombinant plasmids were propagated in *E*. *coli* XL-blue and transferred into *S*. *thermophilus* by electroporation.

### Plasmid curing.

*S. thermophilus* strains were cured of their plasmids by protoplast-induced curing essentially as described by Gasson (1983).

### References

Chopin, A. M.; Chopin, M. C.,Moille-Batt, A. &Langella, P. (1984). Two plasmid-determined restriction and modification systems in Streptococus lactis. Plasmid 11, 260-263
Gasson, J. M. (1983). Plasmid complement of Streptococcus lactis NCDO 712 and other lactic streptococci afterprotoplast-induced curing. J. Bacteriol. 154,1-9
Holo, H. & Nes, I. F. (1989). High-frequency transformation, by electroporation, of Lactococcuslactis subsp. cremoris grown with glycine inosmotically stabilized media. Appl. Environ. Microbiol. 55,3119-3123.
Janzen, T. , Kleinschmidt, J. , Neve, H. & Geis, A. (1992). Sequencing and characterizationof pSTl, a cryptic plasmid from Streptococcus thermophilus. FEMS Microbiol Lett 95,175-180
Krusch, U. , Neve, H. , Luschei, B. & Teuber, M. (1987). Characterization of virulent bacteriophages of Streptococcus salivarius subsp. thermophilus by host specificity and electron microscopy. Kieler Milchwirtsch Forsch Ber 39,155-167
Mohamed, H. A. M. I. (2002). New techniques for food biotechnology. PhD-Thesis, University Kiel, Germany
O'Sullivan, T. , van Sinderen, D. & Fitzgerald, G. (1999). Structural and functional analysisof pCI65st, a 6.5 kb plasmid from Streptococcus thermophilus NDI-6. Microbiology 145,127-134
Sambrook, J. , Fritsch, E. F. & Maniatis, T. (1989). Molecular cloning: a laboratory manual,2nd ed. Cold Spring Harbor Laboratory, Cold Spring Harbor, N. Y.
Somkuti, G. A. , Solaiman, D. K. Y. &Steinberg, D. H. (1998). Structural and functional propertiesofthe hspl6. 4-bearing plasmidpER341 in Streptococcus thermophilus. Plasmid 40, 61-72.
Gonzalez-Marquez, et al., (1997) Microbiology, vol. 143: 1587-1594.

**Table 1: Bacterial strains and bacteriophage**

| Bacterial strains or phage | Relevant characteristics | Source of reference |
|---|---|---|
| *S. thermophilus* | | |
| S4 | pSt04, thermoresistant | BAFM^{a)} |
| S4-1 | plasmid-free S4 | this study |
| S4-1 (p99-17-2) | thermoresistant | this study |
| ER1 | pER 1-2 (shsp), pER 1-2 | BAFM |
| ER1-2 | pER1-1 removed | this study |
| S8 | pSt08 | BAFM |
| S8 (pSt04) | thermoresistant | this study |
| S8 (p99-17-2) | thermoresistant | this study |
| S11 | plasmid-free | Nestle, Vevey, Switzerland |
| S 11 (p99-17-2) | thermoresistant | this study |

| *L. bulgaricus* | | |
|---|---|---|
| 92063 | industrial strain | BAFM |
| HM | industrial strain | BAFM |

| *L. lactis* | | |
|---|---|---|
| MG1363 | plasmid free *L. lactis* 712 | Gasson, 1983 |
| MG1363 (p99-17-2) | thermoresistant | this study |
| IL1403 | plasmid free | Chopin et al., 1984 |
| IL1403 (p99-17-2) | thermoresistant | this study |
| Bu2-60 | plasmid free | BAFM |
| Bu2-60 (p99-17-2) | thermoresistant | this study |
| Bacteriophages s8 | small-isometric headed | BAFM |

| | | |
|---|---|---|
| ^{a)} strain collection of the Institute of Microbiology, Bundesanstalt für Milchforschung, Kiel, Germany | | |

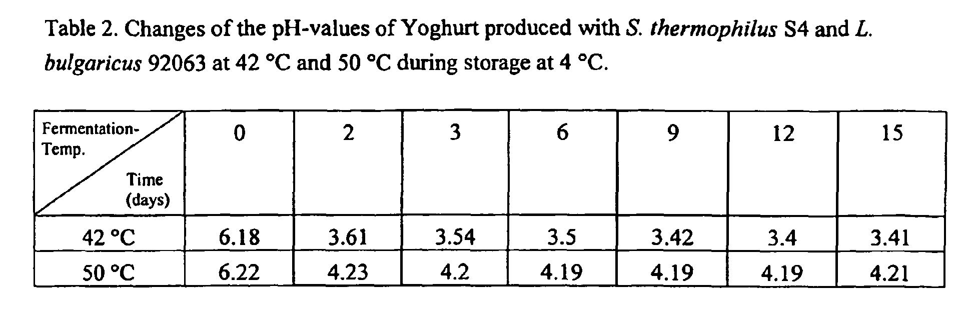

## Claims

1. A method of preparing a milk product, the method comprising the steps of:
combining milk with a lactic acid bacterium capable of expressing a small heat shock protein, and culturing the milk/bacterium mixture at a temperature higher than a regular fermentation temperature and/or at a salt concentration higher than a regular fermentation salt concentration wherein the milk product has milder taste characteristics when compared to a milk product produced with the corresponding lactic acid bacteria which do not express small heat shock proteins.

2. A method of preparing a milk product according to claim 1, wherein said milder taste characteristics product has a higher pH value when compared to the pH value of the milk product produced with the corresponding bacteria which do not express small heat shock proteins.

3. A method according to claim 1 or 2 wherein the pH of the product is above 4.0.

4. A method according to Claims 1 , 2 or 3 , wherein the bacterium comprises a mesophilic lactic acid bacterium and/or a thermophilic lactic acid bacterium.

5. A method according to Claim 4, wherein the method comprises combining milk with a first and a second lactic acid bacterium, in which each of the first and/or second lactic acid bacteria is capable of expressing a small heat shock protein, and culturing the milk/bacterium mixture as specified.

6. A method according to Claim 5, wherein each of said first lactic acid bacterium and said second lactic acid bacterium comprise a mesophilic bacterium.

7. A method according to Claim 6, wherein the milk is combined with a starter culture comprising a first mesophilic lactic acid bacterium prior to the addition of a second mesophilic lactic acid bacterium.

8. A method according to Claim 5, wherein each of said first lactic acid bacterium and said second lactic acid bacterium comprises a thermophilic bacterium.

9. A method according to Claim 8, wherein the milk is combined with a starter culture comprising a first thermophilic lactic acid bacterium prior to the addition of a second thermophilic lactic acid bacterium.

10. A method according to Claim 5 wherein the first lactic acid bacterium comprises a mesophilic lactic acid bacterium and the second lactic acid bacterium comprises a thermophilic lactic acid bacterium.

11. A method according to Claim 10, wherein the milk is combined with a starter culture comprising a mesophilic lactic acid bacterium prior to the addition of a thermophilic lactic acid bacterium.

12. A method according to Claim 11, wherein the milk is combined with a starter culture comprising a thermophilic lactic acid bacterium prior to the addition of a mesophilic lactic acid bacterium.

13. A method according to any one of claims 7 or 9 wherein the starter culture comprising said first lactic bacterium and said second lactic acid bacterium are added to the milk at the same time.

14. A method according to any one of claims 10-12 wherein the starter culture and the second culture are added at the same time.

15. A method according to any one of the preceding claims, wherein said mesophilic bacterium is independently selected from a group consisting of: *Lactococcus* spp, *Leuconostoc* spp, *Lactococcus lactis, Lactococcus lactis* subspecies *cremoris,* and *Lactacoccus lactis* subspecies *lactis.*

16. A method according to any one of the preceding claims, wherein said thermophilic bacterium is independently selected from a group consisting of: *Streptococcus* spp, *Bifidobacterium* spp, *Pediococcus* spp, *Lactobacillus* spp, *Streptococcus thermophilus*, *Lactobacillus debrueckii* or *Lactobacillus debrueckii* subspecies *bulgaricus.*

17. A method according to any one of claims 8 to 14, wherein said first thermophilic bacterium is *Lactobacillus debrueckii* or *Lactobacillus debrueckii* subspecies *bulgaricus,* and said second thermophilic bacterium is *Streptococcus thermophilus.*

18. A method according to any one of the preceding claims, wherein said mesophilic lactic acid bacterium is capable of fermenting milk at up to 43°C.

19. A method according to any one of the preceding claims, wherein said thermophilic lactic acid bacterium is capable of fermenting milk at up to 55°C.

20. A method according to any one of the preceding claims, wherein the method comprises culturing the milk/bacterium mixture at lower pH range than the pH range for culturing milk with the corresponding bacteria which do not express small heat shock proteins.

21. A method according to Claim 20, wherein the lactic acid bacteria remain viable at pH in the range of between 3.9 and 4.5.

22. A method according to Claim 20 or 21, wherein at pH 3.9, at least 10⁶ of the lactic acid bacteria in the culture remain viable when stored for 15 days at 4°C.

23. A method according to any one of the preceding claims, wherein the lactic acid bacterium comprises a recombinant lactic acid bacterium, which has been engineered to express a heat shock protein at a higher level than a corresponding unmodified bacterium.

24. A method according to any one of claims 1-23, wherein the lactic acid bacterium is a recombinant bacterium which has been engineered to express a heat shock protein at a higher level than a corresponding lactic acid bacterium which do not express said heat shock proteins.

25. A method according to claim 24 wherein the recombinant bacterium is capable of carrying out milk fermentation at temperatures and/or salt concentrations higher than regular fermentation temperatures and/or salt concentration.

26. A method of preparing a milk product, the method comprising the steps of:
combining milk with a lactic acid bacterium capable of expressing a heat shock protein, and culturing the milk/bacterium mixture at a temperature higher than a regular fermentation temperature and/or at a salt concentration higher than a regular fermentation salt concentration wherein the milk product is selected from a group consisting of acid curd cheese, hard cheese, semi-hard cheese like mozzarella, fresh cheese, quark, butter milk, Swiss type cheese or cottage cheese.

27. A milk product prepared by a method according to any of Claims 1 to 26.

28. A composition comprising a recombinant lactic acid bacterium according to any of Claims 23-25 together with milk or a milk product.

## Patentansprüche

1. Verfahren zum Herstellen eines Milchprodukts, wobei das Verfahren die Schritte umfasst:
Vermengen von Milch mit einem Milchsäurebakterium, das ein kleines Hitzeschockprotein exprimieren kann, und Kultivieren der Milch/Bakterium-Mixtur bei einer Temperatur, die höher als die reguläre Fermentationstemperatur ist und/oder bei einer Salzkonzentration, die höher als die reguläre Fermentationssalzkonzentration ist, wobei das Milchprodukt mildere Geschmackseigenschaften verglichen mit einem Milchprodukt hat, das mit dem entsprechenden Milchsäurebakterium, das keine kleinen Hitzeschockproteine exprimiert, hergestellt wurde.

2. Verfahren zum Herstellen eines Milchprodukts nach Anspruch 1, wobei das Produkt mit milderen Geschmackseigenschaften einen höheren pH-Wert verglichen mit dem pH-Wert des Milchprodukts hat, das mit dem entsprechenden Milchsäurebakterium, das keine kleinen Hitzeschockproteine exprimiert, hergestellt wurde.

3. Verfahren nach Anspruch 1 oder 2, wobei der pH-Wert des Produkts größer als 4,0 ist.

4. Verfahren nach den Ansprüchen 1, 2 oder 3, wobei das Bakterium ein mesophiles Milchsäurebakterium und/oder ein thermophiles Milchsäurebakterium ist.

5. Verfahren nach Anspruch 4, wobei das Verfahren das Vermengen von Milch mit einem ersten und einem zweiten Milchsäurebakterium, bei denen das erste und/oder das zweite Milchsäurebakterium ein kleines Hitzeschockprotein exprimieren kann und das Kultivieren der Milch/Bakterium-Mixtur wie genannt umfasst.

6. Verfahren nach Anspruch 5, wobei sowohl das erste Milchsäurebakterium als auch das zweite Milchsäurebakterium ein mesophiles Bakterium ist.

7. Verfahren nach Anspruch 6, wobei die Milch vor Zugabe eines zweiten mesophilen Milchsäurebakteriums mit einer Starterkultur vermengt wird, die ein erstes mesophiles Milchsäurebakterium umfasst.

8. Verfahren nach Anspruch 5, wobei sowohl das erste Milchsäurebakterium als auch das zweite Milchsäurebakterium ein thermophiles Bakterium ist.

9. Verfahren nach Anspruch 8, wobei die Milch vor Zugabe eines zweiten thermophilen Milchsäurebakteriums mit einer Starterkultur vermengt wird, die ein erstes thermophiles Milchsäurebakterium umfasst.

10. Verfahren nach Anspruch 5, wobei das erste Milchsäurebakterium ein mesophiles Milchsäurebakterium umfasst und das das zweite Milchsäurebakterium ein thermophiles Milchsäurebakterium umfasst.

11. Verfahren nach Anspruch 10, wobei die Milch vor Zugabe eines thermophilen Milchsäurebakteriums mit einer Starterkultur vermengt wird, die ein mesophiles Milchsäurebakterium umfasst.

12. Verfahren nach Anspruch 11, wobei die Milch vor Zugabe eines mesophilen Milchsäurebakteriums mit einer Starterkultur vermengt wird, die ein thermophiles Milchsäurebakterium umfasst.

13. Verfahren nach einem der Ansprüche 7 oder 9, wobei die Starterkultur, die das erste Milchsäurebakterium umfasst, und das zweite Milchsäurebakterium zur selben Zeit zur Milch gegeben werden.

14. Verfahren nach einem der Ansprüche 10-12, wobei die Starterkultur und die zweite Kultur zur selben Zeit hinzugefügt werden.

15. Verfahren nach einem der vorhergehenden Ansprüche, wobei das mesophile Bakterium unabhängig ausgewählt wurde aus der Gruppe bestehend aus: *Lactococcus* spp., *Leuconostoc* spp., *Lactococcus lactis, Lactococcus lactis* subspecies *cremoris* und *Lactococcus lactis* subspecies *lactis.*

16. Verfahren nach einem der vorhergehenden Ansprüche, wobei das thermophile Bakterium unabhängig ausgewählt wurde aus der Gruppe bestehend aus:
*Streptococcus* spp., *Bifidobacterium* spp., *Pediococcus* spp., *Lactobacillus* spp., *Streptococcus thermophilus, Lactobacillus debrueckii* oder *Lactobacillus debrueckii* subspecies *bulgaricus.*

17. Verfahren nach einem der Ansprüche 8 bis 14, wobei das erste thermophile Bakterium *Lactobacillus debrueckii* oder *Lactobacillus debrueckii* subspecies *bulgaricus* und das zweite thermophile Bakterium *Streptococcus thermophilus* ist.

18. Verfahren nach einem der vorhergehenden Ansprüche, wobei das mesophile Milchsäurebakterium Milch bei über 43 °C fermentieren kann.

19. Verfahren nach einem der vorhergehenden Ansprüche, wobei das thermophile Milchsäurebakterium Milch bei über 55 °C fermentieren kann.

20. Verfahren nach einem der vorhergehenden Ansprüche, wobei das Verfahren das Kultivieren der Milch/Bakterium-Mixtur in einem geringeren pH-Bereich als dem pH-Bereich zum Kultivieren von Milch mit entsprechenden Bakterien umfasst, die kleine Hitzeschockproteine nicht exprimieren.

21. Verfahren nach Anspruch 20, wobei das Milchsäurebakterium in einem pH-Bereich von zwischen 3,9 und 4,5 lebensfähig verbleibt.

22. Verfahren nach Anspruch 20 oder 21, wobei bei pH 3,9 wenigstens 10⁶ der Milchsäurebakterien in der Kultur lebensfähig verbleiben, wenn diese für 15 Tage bei 4 °C gelagert wird.

23. Verfahren nach einem der vorhergehenden Ansprüche, wobei das Milchsäurebakterium ein rekombinantes Milchsäurebakterium umfasst, das manipuliert wurde, ein Hitzeschockprotein mit einem höheren Maß zu exprimieren als ein entsprechendes unmodifiziertes Bakterium.

24. Verfahren nach einem der Ansprüche 1-23, wobei das Milchsäurebakterium ein rekombinantes Bakterium ist, das manipuliert wurde, ein Hitzeschockprotein mit einem höheren Maß zu exprimieren als ein entsprechendes Milchsäurebakterium, das diese Hitzeschockproteine nicht exprimiert.

25. Verfahren nach Anspruch 24, wobei das rekombinante Bakterium die Fermentation von Milch bei Temperaturen und/oder Salzkonzentrationen ausführen kann, die höher sind als die regulären Fermentationstemperaturen und/oder Salzkonzentrationen.

26. Verfahren zum Herstellen eines Milchprodukts, wobei das Verfahren die Schritte umfasst:
Vermengen von Milch mit einem Milchsäurebakterium, das ein Hitzeschockprotein exprimieren kann, und Kultivieren der Milch/Bakterium-Mixtur bei einer Temperatur, die höher als die reguläre Fermentationstemperatur, und/oder eines Salzkonzentration, die höher als eine reguläre Fermentationssalzkonzentration ist, wobei das Milchprodukt ausgewählt ist aus der Gruppe bestehend aus Sauermilchkäse, Hartkäse, halbharter Käse wie Mozzarella, Frischkäse, Quark, Buttermilch, Schweizer Käse oder Hüttenkäse.

27. Milchprodukt, das mittels eines Verfahrens nach einem der Ansprüche 1 bis 26 hergestellt wurde.

28. Zusammensetzung umfassend ein rekombinantes Milchsäurebakterium nach einem der Ansprüche 23-25 zusammen mit Milch oder einem Milchprodukt.

## Revendications

1. Procédé de préparation d'un produit laitier, le procédé comprenant les étapes consistant à : combiner du lait avec une bactérie d'acide lactique capable d'exprimer une petite protéine de choc thermique et mettre en culture le mélange lait/bactérie à une température supérieure à une température de fermentation normale et/ou à une concentration de sel supérieure à une concentration de sel de fermentation normale, le produit laitier ayant une caractéristique de goût plus doux par comparaison à un produit laitier fabriqué avec les bactéries d'acides lactiques correspondantes qui n'expriment pas de petites protéines de choc thermique.

2. Procédé de préparation d'un produit laitier selon la revendication 1, dans lequel ledit produit à caractéristique de goût plus doux présente une valeur de pH plus élevée par comparaison à la valeur de pH du produit laitier fabriqué avec les bactéries correspondantes qui n'expriment pas de petites protéines de choc thermique.

3. Procédé selon la revendication 1 ou 2, dans lequel le pH du produit est supérieur à 4,0.

4. Procédé selon la revendication 1, 2 ou 3, dans lequel la bactérie comprend une bactérie d'acide lactique mésophile et/ou une bactérie d'acide lactique thermophile.

5. Procédé selon la revendication 4, dans lequel le procédé comprend la combinaison du lait avec une première et une deuxième bactéries d'acides lactiques, dans lequel chacune des première ou deuxième bactéries d'acides lactiques est capable d'exprimer une petite protéine de choc thermique et la mise en culture du mélange lait/bactérie comme spécifié.

6. Procédé selon la revendication 5, dans lequel chacune de ladite première bactérie d'acide lactique et de ladite deuxième bactérie d'acide lactique comprend une bactérie mésophile.

7. Procédé selon la revendication 6, dans lequel le lait est combiné à une culture starter ou de démarrage comprenant une première bactérie d'acide lactique mésophile avant l'addition d'une deuxième bactérie d'acide lactique mésophile.

8. Procédé selon la revendication 5, dans lequel chacune de ladite première bactérie d'acide lactique et de ladite deuxième bactérie d'acide lactique comprend une bactérie thermophile.

9. Procédé selon la revendication 8, dans lequel le lait est combiné à une culture starter ou de démarrage comprenant une première bactérie d'acide lactique thermophile avant l'ajout d'une deuxième bactérie d'acide lactique thermophile.

10. Procédé selon la revendication 5, dans lequel la première bactérie d'acide lactique comprend une bactérie d'acide lactique mésophile et la deuxième bactérie d'acide d'acide lactique comprend une bactérie d'acide lactique thermophile.

11. Procédé selon la revendication 10, dans lequel le lait est combiné à une culture starter comprenant une bactérie d'acide lactique mésophile avant l'ajout d'une bactérie d'acide lactique thermophile.

12. Procédé selon la revendication 11, dans lequel le lait est combiné à une culture starter ou de démarrage comprenant une bactérie d'acide lactique thermophile avant l'ajout d'une bactérie d'acide lactique mésophile.

13. Procédé selon l'une quelconque des revendications 7 ou 9, dans lequel les cultures starters ou de démarrage comprenant ladite première bactérie d'acide lactique et ladite deuxième bactérie d'acide lactique sont ajoutées au lait en même temps.

14. Procédé selon l'une quelconque des revendications 10 à 12, dans lequel la culture starter ou de démarrage et la deuxième culture sont ajoutées en même temps.

15. Procédé selon l'une quelconque des revendications précédentes, dans lequel ladite bactérie mésophile est sélectionnée indépendamment à partir d'un groupe constitué de *Lactococcus* spp, *Leuconostoc* spp, *Lactococcus lactis, Lactococcus lactis* sous-espèce *cremoris,* et *Lactacoccus lactis* sous-espèce *lactis.*

16. Procédé selon l'une quelconque des revendications précédentes, dans lequel ladite bactérie thermophile est sélectionnée indépendamment à partir d'un groupe constitué de : *Streptococcus* spp, *Bifidobacterium* spp, *Pediococcus* spp, *Lactobacillus* spp, *Streptococcus thermophilus, Lactobacillus delbrueckii* ou *Lactobacillus delbrueckii* sous-espèce *bulgaricus.*

17. Procédé selon l'une quelconque des revendications 8 à 14, dans lequel ladite première bactérie thermophile est le *Lactobacillus delbrueckii* ou le *Lactobacillus delbrueckii* sous-espèce *bulgaricus,* et ladite deuxième bactérie thermophile est le *Streptococcus thermophilus.*

18. Procédé selon l'une quelconque des revendications précédentes, dans lequel ladite bactérie lactique mésophile est capable de faire fermenter le lait jusqu'à 43 °C.

19. Procédé selon l'une quelconque des revendications précédentes, dans lequel ladite bactérie lactique thermophile est capable de faire fermenter le lait jusqu'à 55 °C.

20. Procédé selon l'une quelconque des revendications précédentes, dans lequel le procédé comprend la mise en culture du mélange lait/bactérie à une plage de pH inférieure à la plage de pH pour la mise en culture avec les bactéries correspondantes qui n'expriment pas des petites protéines de choc thermique.

21. Procédé selon la revendication 20, dans lequel les bactéries lactiques restent viables à un pH situé dans la plage comprise entre 3,9 et 4,5.

22. Procédé selon la revendication 20 ou 21, dans lequel, au pH 3,9, au moins 10⁶ des bactéries d'acide lactique dans la culture restent viables lorsqu'elles sont stockées pendant 15 jours à 4 °C.

23. Procédé selon l'une quelconque des revendications précédentes, dans lequel la bactérie d'acide lactique comprend une bactérie d'acide lactique recombinante, qui a été créée pour exprimer une protéine de choc thermique à un niveau plus élevé qu'une bactérie non modifiée correspondante.

24. Procédé selon l'une quelconque des revendications 1 à 23, dans lequel la bactérie d'acide lactique est une bactérie recombinante qui a été créée pour exprimer une protéine de choc thermique à un niveau plus élevé qu'une bactérie d'acide lactique correspondante qui n'exprime pas lesdites protéines de choc thermique.

25. Procédé selon la revendication 24, dans lequel la bactérie recombinante est capable d'exécuter la fermentation du lait à des températures et/ou des concentrations de sel supérieures aux températures de fermentation normales et/ou à la concentration de sel normale.

26. Procédé de préparation d'un produit laitier, le procédé comprenant les étapes consistant à : combiner du lait avec une bactérie d'acide lactique capable d'exprimer une protéine de choc thermique et mettre en culture le mélange lait/bactérie à une température supérieure à une température de fermentation normale et/ou à une concentration de sel supérieure à une concentration de sel de fermentation normale, où le produit laitier est sélectionné parmi un groupe constitué d'un fromage de lait caillé lactique, un fromage dur, un fromage demi-dur tel que la mozzarella, un fromage frais, de la caillebotte, du babeurre, du fromage de type petit suisse ou du fromage blanc.

27. Produit laitier préparé par un procédé selon l'une quelconque des revendications 1 à 26.

28. Composition comprenant une bactérie d'acide lactique recombinante conforme à l'une quelconque des revendications 23 à 25 en même temps que du lait ou un produit laitier.
